# EUROPÄISCHE PATENTSCHRIFT

(11) **EP 2 392 272 B1**
(45) Veröffentlichungstag und Bekanntmachung des Hinweises auf die Patenterteilung: **06.07.2016**
(21) Anmeldenummer: 11165953.8
(22) Anmeldetag: 13.05.2011
(51) Int. Cl.: A61B 17/29, A61B 17/00, A61B 18/14

(54) **Medizinisches Greifwerkzeug**
Medical gripping tool
Outil de préhension médical

(30) Priorität: 01.06.2010 DE 102010022431
(43) Veröffentlichungstag der Anmeldung: 07.12.2011
(73) Patentinhaber: Karl Storz GmbH & Co. KG, 78532 Tuttlingen (DE)
(72) Erfinder: Staud, Ralf, 78532, Tuttlingen (DE)

(56) Entgegenhaltungen:
- EP-A1- 1 201 198
- DE-A1-102007 026 721
- DE-A1-102007 050 018
- DE-U1- 20 318 845
- US-A- 4 274 413
- US-A1- 2003 171 748

## Beschreibung

Die vorliegende Erfindung bezieht sich auf ein medizinisches Greifwerkzeug, insbesondere für mikroinvasive Eingriffe, mit einem Kunststoffkörper und einer Metallschicht an einer Oberfläche des Kunststoffkörpers, wobei der Kunststoffkörper dazu ausgebildet ist, bei seiner vorgesehenen Verwendung verformt zu werden. Außerdem bezieht sich die vorliegende Erfindung auf ein Verfahren zum Handhaben eines medizinischen Objekts, insbesondere bei einem mikroinvasiven Eingriff, mit folgenden Schritten: Positionieren eines medizinischen Greifwerkzeugs mit einem Kunststoffkörper und einer Metallschicht an einer Oberfläche des Kunststoffkörpers an dem medizinischen Gegenstand; Verformen des medizinischen Greifwerkzeugs, um den medizinischen Gegenstand zu greifen, wobei der Kunststoffkörper verformt wird. Ein derartiges medizinisches Greifwerkzeug gemäß dem Oberbegriff des Anspruchs 1, und ein derartiges Verfahren sind aus der DE 20 2007 009 310 U1 bekannt.

Bei medizinischen Eingriffen können Gefäße, Organe, Gewebe oder andere medizinische Objekte oft nicht unmittelbar manuell gegriffen bzw. gehalten werden. Dies gilt insbesondere für mikroinvasive Eingriffe. In diesen Fällen werden Greifwerkzeuge verwendet.

Diese stellen zumindest im weiteren Sinne Zangen dar. Für verschiedene Anwendungen, insbesondere Gewebe unterschiedlicher mechanischer Eigenschaften und unterschiedlicher Empfindlichkeit steht ein breites Spektrum unterschiedlicher medizinischer Greifwerkzeuge zur Verfügung, um ein gleichzeitig sicheres und schonendes bzw. atraumatisches Greifen bzw. Halten zu ermöglichen.

Neuere Entwicklungen zielen auf die Verwendung von Kunststoff für medizinische Greifwerkzeuge. Insbesondere im Spritzgussverfahren können Greifwerkzeuge aus Kunststoff schnell und in großer Anzahl kostengünstig hergestellt werden. Die vor allem im Vergleich zu herkömmlich oft verwendeten Metallen höhere Elastizität von Kunststoff ermöglicht teilweise eine fundamental andere Gestaltung. Beispielsweise können Festkörpergelenke vorgesehen werden.

In der DE 10 2007 026 721 A1 und in der DE 10 2010 009 259.2 sind formadaptive medizinische Greifwerkzeuge beschrieben, die auf dem Flossenstrahlen-Effekt (englisch: Fin-Ray-Effect) beruhen. Die genannte DE 10 2007 026 721 A1 offenbart auch eine Greifschicht, die mit nicht näher spezifizierten Mikro-oder Nanopartikeln beschichtet ist. Die DE 10 2007 050 018 A1 offenbart ein medizinisches Greifwerkzeug mit Federelementen in Fluidkammern oder zur Abstützung von Greifflächen. DE 698 32 497 T2 offenbart ein medizinisches Greifwerkzeug mit einer Greifschicht, die mit nicht miteinander verbundenen Metall-Körnchen beschichtet ist.

Für manche Anwendungen weisen jedoch medizinische Greifwerkzeuge aus Kunststoff eine zu geringe mechanische Festigkeit auf. Beispielsweise können medizinische Greifwerkzeuge aus Kunststoff, wenn sie ein Drehmoment auf das gehaltene medizinische Objekt übertragen sollen, eine unerwünschte Verformung, insbesondere Torsion, zeigen, die nicht oder nur mit hohem konstruktivem Aufwand vermindert oder beseitigt werden kann.

In EP 1 201 198 A1 ist ein Katheter mit mehrlagigen Dünnfilmelektroden auf einem flexiblen Polymerkörper beschrieben, jedoch kein Greifwerkzeug.

In DE 203 18 845 U1 ist ein weiteres formadaptives Greifwerkzeug mit einem Stab- oder Fachwerk beschrieben, jedoch keine Metallschicht auf einem Kunststoffkörper.

In US 2003/0171748 A1 ist ein elektrochirurgisches Instrument mit einem kinematischen Gelenk beschrieben, jedoch kein Greifwerkzeug mit einem bei der vorgesehenen Verwendung sich verformenden Kunststoffkörper.

In US 4,274,413 ist eine Enthaarungspinzette beschrieben.

Eine Aufgabe der vorliegenden Erfindung besteht darin, ein verbessertes medizinisches Greifwerkzeug und ein verbessertes Verfahren zum Handhaben eines medizinischen Objekts zu schaffen.

Diese Aufgabe wird durch den Gegenstand des unabhängigen Anspruchs gelöst.

Weiterbildungen sind in den abhängigen Ansprüchen angegeben.

Ausführungsformen der vorliegenden Erfindung beruhen auf der Idee, ein medizinisches Greifwerkzeug mit einem Kunststoffkörper und einer Metallschicht auf einer Oberfläche des Kunststoffkörpers zu schaffen. Metallschichten an Oberflächen von Kunststoffkörpern sind aus verschiedenen Bereichen der Technik bereits bekannt. Sie dienten dort ursprünglich hauptsächlich dekorativen Zwecken. Die kostengünstige Herstellung von Spritzgussteilen soll mit der Anmutung eines Metallbauteils verbunden werden. Eine mechanische Belastung führt in der Regel zum Reißen und Abplatzen der Metallschicht. Von einem medizinischen Instrument sich lösende Partikel stellen jedoch ein erhebliches Gesundheitsrisiko für Patienten dar. Ein medizinisches Greifwerkzeug mit einem Kunststoffkörper, der ausgebildet ist, um bei der vorgesehenen Verwendung elastisch verformt zu werden, und einer Metallschicht an einer Oberfläche des Kunststoffkörpers ist deshalb herkömmlich undenkbar.

Es wurde jedoch erkannt, dass neuere Technologien zwischenzeitlich eine Entwicklung und Ausbildung des Kunststoffkörpers und der Metallschicht an seiner Oberfläche ermöglichen, die ein Reißen und Abplatzen der Metallschicht auch bei wiederholter erheblicher elastischer oder plastischer Verformung des Kunststoffkörpers und plastischer Verformung der Metallschicht mit ausreichender Sicherheit verhindern können. Damit können erstmals die Vorteile der geringen Herstellungskosten und der Elastizität von Kunststoffkörpern für medizinische Greifwerkzeuge genutzt werden.

Mit einer Verformung des Kunststoffkörpers ist eine nicht nur geringfügige Verformung gemeint, wie sie insbesondere an Festkörpergelenken auftritt. Diese Verformung kann rein elastisch oder teilweise elastisch und teilweise plastisch sein. Eine geringfügige Verformung, wie sie bei jedem realen Bauteil bei Belastung unvermeidbar ist, ist nicht gemeint.

Der Kunststoffkörper kann kostengünstig herstellbar sein, beispielsweise im Spritzgussverfahren. Auch die Metallschicht an seiner Oberfläche kann, je nach ihren Eigenschaften und ihrer Herstellung, kostengünstig herstellbar sein. Das medizinische Greifwerkzeug kann damit insgesamt so niedrige Herstellungskosten aufweisen, dass es zur einmaligen Verwendung und anschließenden Entsorgung vorgesehen sein kann. Damit können Aufwand und Kosten für Reinigung und Sterilisierung des Greifwerkzeugs nach jeder Verwendung entfallen.

Aufgrund der elastischen Verformbarkeit des Kunststoffkörpers können beispielsweise herkömmliche Gelenke mit Wellen und Lagern entfallen, die bei herkömmlichen Greifwerkzeugen aus Metall einen hohen Herstellungsaufwand hervorrufen. Aufgrund hoher Herstellungskosten herkömmlicher Greifwerkzeuge aus Metall müssen diese vielfach verwendet werden und können nicht bereits nach einer Verwendung verworfen bzw. entsorgt werden. Gerade herkömmliche Gelenke mit Wellen und Lagern weisen jedoch Verschleiß auf und können durch Verschmutzung schwergängig oder gehemmt werden. Dies kann oft nur durch hohen Aufwand bei Entwurf und Herstellung vermieden werden.

Die Metallschicht an der Oberfläche des Kunststoffkörpers ermöglicht definierte mechanische Eigenschaften. Insbesondere ist mit der Metallschicht eine hohe mechanische Festigkeit erzielbar, die beispielsweise auch bei Wirken eines Drehmomentes eine Torsion des Greifwerkzeugs auf ein hinnehmbares Maß reduzieren kann. Eine ausreichende mechanische Festigkeit ist dabei auch bei deutlich geringeren Querschnitten am Kunststoffkörper erzielbar. Das medizinische Greifwerkzeug kann deshalb unter Umständen bei.gleicher Haltkraft deutlich schlanker oder kleiner ausgeführt werden.

Bei dem erfindungsgemäßen medizinischen Greifwerkzeug, ist die Metallschicht dazu ausgebildet, bei der vorgesehenen Verwendung plastisch verformt zu werden.

Eine wiederholte plastische Verformung eines Metalls kann zu dessen Sprödbruch führen. Dies gilt jedoch in deutlich reduziertem Maße bei einer Metallschicht mit einer Korngröße von 100 nm oder weniger, und in noch geringerem Maße bei Korngrößen von 30 nm oder weniger (insbesondere 10 nm bis 20 nm). Somit können das medizinische Greifwerkzeug, der Kunststoffkörper und die Metallschicht an dessen Oberfläche beispielsweise so dimensioniert bzw. ausgebildet sein, dass bei der vorgesehenen Verwendung beispielsweise im Bereich eines Festkörpergelenks der Kunststoffkörper elastisch und die Metallschicht an seiner Oberfläche plastisch verformt werden. Die Metallschicht kann somit gleichzeitig einerseits die mechanische Festigkeit des medizinischen Greifwerkzeugs erhöhen und andererseits ohne die Gefahr eines Reißens oder Abblättems eine Ausbildung von Festkörpergelenken ermöglichen.

Die Metallschicht kann aus einer einzigen Schicht bestehen oder ein Mehrschichtsystem umfassen. Bewährt haben sich insbesondere Zweischichtsysteme mit Kombinationen und Legierungen von Brom, Cobalt, Molybdän, Nickel, Chrom, Gold, Kohlenstoff, Kupfer, Palladium, Phosphor, Platin, Silber, Schwefel, Titan, Wolfram, Zinn, Eisen sowie Oxiden dieser Elemente. Die Metallschicht bzw. einzelne Schichten eines Mehrschichtsystems können jeweils mikroskopisch homogen sein oder eine Matrix und darin eingelagerte Metall- oder andere.Partikel aufweisen. In diesem Fall weisen die Matrix und die darin eingelagerten Partikel unterschiedliche Materialien auf. Die in die Matrix eingelagerten Partikel weisen insbesondere ebenfalls Größen von höchstens 100 nm oder von höchstens 30 nm (insbesondere 10 nm bis 20 nm) auf. Die Partikel können ebenfalls Kombinationen oder Legierungen der oben genannten Elemente aufweisen.

Bei einem medizinischen Greifwerkzeug, wie es hier beschrieben ist, umfasst der Kunststoffkörper insbesondere.eine erste Backe, eine zweite Backe und ein Festkörpergelenk zwischen der ersten Backe und der zweiten Backe.

Bei dieser Ausführungsform des medizinischen Greifwerkzeugs kommt der bereits erwähnte Vorteil der Elastizität des Kunststoffs zum Tragen. Während herkömmlich zumindest zwei Backen und eine Welle separat herzustellen und anschließend zu montieren wären, ist hier lediglich ein Teil herzustellen, und eine Montage kann entfallen. Die, wie bereits erwähnt, ohnehin vergleichsweise geringen Herstellungskosten können dadurch noch weiter verringert werden. Dies begünstigt eine Ausbildung des medizinischen Greifwerkzeugs für eine einmalige Verwendung und anschließende Entsorgung.

Bei einem medizinischen Greifwerkzeug, wie es hier beschrieben ist, kann zumindest entweder die erste Backe oder die zweite Backe eine Fachwerk-Struktur aufweisen.

Bei einer Fachwerk-Struktur weist eine Backe mehrere Balken oder Stege bzw. balken- oder stegförmige Abschnitte auf. Diese Balken und Stege können jeweils gerade oder gekrümmt sein und konstante oder variierende Querschnitte aufweisen. Jeweils drei oder vier Balken oder Stege können ein Dreieck bzw. ein Viereck bilden.

Mittels der Fachwerk-Struktur sind definierte und innerhalb weiter Grenzen einstellbare mechanische Eigenschaften bei vergleichsweise geringer Masse erzielbar, insbesondere eine besonders hohe Steifheit oder eine definierte Elastizität. Sowohl die Steifheit als auch die Elastizität der Fachwerk-Struktur können mittels der Metallschicht gezielt beeinflusst werden. Die Metallschicht kann dazu in unterschiedlichen Bereichen des medizinischen Greifwerkzeugs unterschiedliche Schichtdicken und/oder unterschiedliche Materialien und/oder unterschiedliche Schichtaufbauten aufweisen. Ferner können einzelne Bereiche beschichtet werden, um deren Steifheit zu erhöhen, während einer oder mehrere andere Bereiche nicht beschichtet werden, um die Elastizität des Kunststoffkörpers lokal ungemindert zu erhalten.

Bei einem medizinischen Greifwerkzeug, wie es hier beschrieben ist, kann zumindest entweder die erste Backe oder die zweite Backe ausgebildet sein, um sich beim Greifen eines Gegenstands entsprechend dem Flossenstrahlen-Effekt zu verformen.

Der Flossenstrahlen-Effekt ist auch unter der englischen Bezeichnung Fin-Ray-Effect bekannt. Ähnliche medizinische Greifwerkzeuge sind in der DE 10 2007 026 721 A1 und in der DE 10 2010 009 259.2 beschrieben. Gerade bei einem Greifwerkzeug, das auf dem Flossenstrahlen-Effekt beruht, sind definierte und differenzierte sowie von Ort zu Ort innerhalb des Greifwerkzeugs variierende mechanische Eigenschaften von Vorteil.

Schon eine hinsichtlich ihrer Dicke und ihrer sonstigen Eigenschaften homogene Metallschicht am Kunststoffkörper kann in diesem Zusammenhang eine deutliche Verbesserung bewirken. Das Metall der Metallschicht weist in der Regel eine deutlich geringere Elastizität auf als der Kunststoff des Kunststoffkörpers. Da die Metallschicht an der Oberfläche des Kunststoffkörpers beispielsweise im Fall eines Stegs oder Balkens den maximalen Abstand von der neutralen Faser des Balkens hat, hat sie großen Einfluss auf die Biegeelastizität des Balkens. Der Querschnitt des Balkens, insbesondere der Abstand der Oberfläche des Balkens von der neutralen Faser hat deshalb einen ausgeprägteren Einfluss auf die Biegeelastizität des Balkens, wenn an der Oberfläche die beschriebene Metallschicht angeordnet ist. Beispielsweise im Fall von Festkörpergelenken mit lokal reduziertem Querschnitt ist die elastische Verformung deshalb noch ausgeprägter im Bereich des reduzierten Querschnitts lokalisiert, wenn an der Oberfläche eine Metallschicht vorgesehen ist. Die Möglichkeit, die Verformung an einem Festkörpergelenk mittels der Metallschicht noch stärker zu lokalisieren, erlaubt eine noch bessere Definition der kinematischen Eigenschaften des medizinischen Greifwerkzeugs.

Eine weitere Verbesserung ist möglich, wenn die Metallschicht eine variierende Dicke oder eine variierende andere Eigenschaft aufweist oder beispielsweise im Bereich der Festkörpergelenke gar nicht vorgesehen ist.

Bei einem medizinischen Greifwerkzeug, wie es hier beschrieben ist, weist die Metallschicht insbesondere eine mittlere Korngröße von höchstens 100 nm auf.

Die mittlere Korngröße ist die mittlere Größe der Kristallite der polykristallinen Metallschicht. Da Kristallite der polykristallinen Metallschicht allenfalls in Ausnahmefällen näherungsweise kugelförmig sind, wird als Größe eines Kristallits der Äquivalentdurchmesser des Kristallits angesehen. Der Äquivalentdurchmesser ist hier die Kantenlänge eines quadratischen Lochs eines Siebs, durch das der Kristallit gerade noch passt.

Bei einer mittleren Korngröße von höchstens 100 nm weisen viele Metalle eine deutlich erhöhte Duktilität auf im Vergleich zu gewöhnlichen Korngrößen, die um mehrere Größenordnungen größer sind. Das Risiko eines Reißens und Abplatzens der Metallschicht ist damit deutlich verringert. Besonders gute Ergebnisse wurden erzielt bei einer mittleren Korngröße von höchstens 30 nm, insbesondere bei mittleren Korngrößen von 10 nm bis 20 nm. Eine mittlere Korngröße von höchstens 100 nm und mehr noch eine mittlere Korngröße von höchstens 30 nm oder von 10 nm bis 20 nm ist insbesondere für die bereits erwähnten Ausbildungen des medizinischen Greifwerkzeugs geeignet, bei denen die Metallschicht bei der vorgesehenen Verwendung plastisch verformt wird. Die besondere Eignung rührt insbesondere daher, dass die besonders hohe Duktilität auch ein Reißen und Abplatzen der Metallschicht bei wiederholter starker Verformung, beispielsweise im Bereich eines Festkörpergelenks, verhindern kann.

Bei einem medizinischen Greifwerkzeug, wie es hier beschrieben ist, kann die Metallschicht an einer Greiffläche des Greifwerkzeugs vorgesehen sein.

Insbesondere ist die Metallschicht ausschließlich oder im Wesentlichen ausschließlich an einer oder an beiden Greifflächen des Greifwerkzeugs vorgesehen. Eine elektrisch leitfähige Metallschicht an einer Greiffläche des Greifwerkzeugs kann eine elektrische. Funktionalität des Greifwerkzeugs ermöglichen. Beispielsweise kann eine hochfrequente Wechselspannung geeigneter Frequenz und Amplitude an die Metallschicht an der Greiffläche des Greifwerkzeugs angelegt werden, um durch Koagulation Gewebe zu veröden oder zu durchtrennten.

Bei einem medizinischen Greifwerkzeug, wie es hier beschrieben ist, kann an einer Greiffläche des Greifwerkzeugs keine Metallschicht oder eine dünnere Metallschicht als an anderen Bereichen der Oberfläche des Greifwerkzeugs vorgesehen sein.

Indem an einer Greiffläche des Greifwerkzeugs keine Metallschicht oder nur eine dünne Metallschicht vorgesehen ist, kann die Elastizität des Kunststoffkörpers im Bereich der Greiffläche vollständig oder weitgehend erhalten bleiben. Dies kann ein schonendes oder atraumatisches Greifen von Gewebe begünstigen. Ferner kann die Reibung zwischen der Kunststoffoberfläche des Kunststoffkörpers und Gewebe höher sein als zwischen einer Metallschicht und Gewebe.

Ein medizinisches Greifwerkzeug, wie es hier beschrieben ist, ist beispielsweise als Nadelhalter ausgebildet. Die Metallschicht ermöglicht eine hinreichend steife Auslegung des Nadelhalters, um die Übertragung großer Kräfte auf die Nadel zu ermöglichen. Gleichzeitig kann eine nicht mit der Metallschicht versehene Greiffläche des Nadelhalters einen guten Kraft- bzw. Reibschluss zwischen dem Nadelhalter bzw. dem Kunststoffkörper einerseits und der Nadel andererseits ermöglichen.

Mit einer dünnen Metallschicht kann die Elastizität des Kunststoffkörpers an der Greiffläche teilweise oder weitgehend erhalten bleiben, wobei gleichzeitig eine elektrische Funktionalität realisierbar ist.

Bei einem medizinischen Greifwerkzeug, wie es hier beschrieben ist, kann an einem Festkörpergelenk des Greifwerkzeugs keine Metallschicht oder eine dünnere Metallschicht als an anderen Bereichen der Oberfläche des Greifwerkzeugs vorgesehen sein.

Wie bereits angedeutet, kann die Metallschicht die Biegelastizität eines, beispielsweise balkenförmigen Abschnitts des Kunststoffkörpers deutlich reduzieren. Indem im Bereich eines Festkörpergelenks keine Metallschicht oder eine dünnere Metallschicht vorgesehen ist als an anderen Bereichen der Oberfläche des Kunststoffkörpers, kann die Elastizität des Kunststoffkörpers im Bereich des Festkörpergelenks in vollem Umfang erhalten bleiben. Da in anderen Bereichen die Metallschicht die Elastizität des Kunststoffkörpers deutlich reduzieren kann, ermöglicht die beschriebene Ausbildung der Metallschicht eine besonders gute Definition bzw. Begrenzung des Festkörpergelenks und damit auch eine besonders definierte Kinematik des medizinischen Greifwerkzeugs.

Ein medizinisches Greifwerkzeug, wie es hier beschrieben ist, kann ferner eine Kontaktierungseinrichtung zum elektrischen Kontaktieren der Metallschicht umfassen, wobei die Metallschicht einen Abschnitt an einer Greiffläche umfasst, wobei der Abschnitt an der Greiffläche elektrisch leitfähig mit der Kontaktierungseinrichtung verbunden ist.

Die Kontaktierungseinrichtung ist beispielsweise eine Lötfahne, ein Steckkontakt oder ein Abschnitt der Metallschicht, der besonders dick ausgebildet sein kann. Der Abschnitt an der Greiffläche ist insbesondere ohne eine Oxidschicht oder eine andere elektrisch isolierende Schicht ausgebildet. Die Kontaktierungseinrichtung und der Abschnitt der Metallschicht an der Greiffläche sind beispielsweise unmittelbar oder mittels einer Leiterbahn oder mittels eines Drahts elektrisch leitfähig miteinander verbunden. Die beschriebene Ausbildung des medizinischen Greifwerkzeugs ermöglicht eine elektrische Funktionalität, beispielsweise das Durchtrennen und/oder Veröden von Gewebe durch Koagulation.

Bei einem medizinischen Greifwerkzeug, wie es hier beschrieben ist, kann die Metallschicht eine Nickelschicht mit einer mittleren Korngröße von höchstens 100 nm und eine Kupferschicht zwischen dem Kunststoffkörper und der Nickelschicht umfassen.

Die Vorteile einer geringen mittleren Korngröße in der Metallschicht wurden bereits beschrieben und gelten insbesondere für eine Nickelschicht. Die Kupferschicht zwischen dem Kunststoffkörper und der Nickelschicht ermöglicht eine besonders dauerhafte und verlässliche Haftung der Nickelschicht an der Oberfläche des Kunststoffkörpers. Die Kupferschicht weist insbesondere eine mittlere Korngröße von höchstens 100 nm auf.

Bei einem Verfahren zum Handhaben eines medizinischen Objekts wird ein medizinischer Greifer mit einem Kunststoffkörper und einer Metallschicht an einer Oberfläche des Kunststoffkörpers an dem medizinischen Objekt positioniert und verformt, um das medizinische Objekt zu greifen, wobei der Kunststoffkörper verformt wird.

Gemäß der vorliegenden Erfindung wird beim Verformen des medizinischen Greifwerkzeugs die Metallschicht plastisch verformt.

Wie bereits beschrieben, wurden metallbeschichtete Kunststoffkörper herkömmlich so ausgebildet, dass bei der vorgesehenen Verwendung eine allenfalls geringfügige elastische Verformung des Kunststoffkörpers auftrat, um ein Reißen und Abplatzen der Metallschicht zu verhindern. Es wurde jedoch erkannt, dass bei einer geeigneten Ausbildung der Metallschicht, insbesondere bei den erwähnten geringen Korngrößen, das medizinische Greifwerkzeug durchaus so ausgestaltet werden kann, dass bei der vorgesehenen Verwendung die Metallschicht sogar plastisch verformt werden kann, ohne ein Reißen oder Abplatzen der Metallschicht zu riskieren.

Der Kunststoffkörper weist beispielsweise ABS (Acrylnitril-Butadien-Styrol), LCP (Flüssigkristallpolymer bzw. Liquid Crystal Polymer), PA (Polyamid), PC (Polycarbonat), PEEK (Polyetheretherketon), PEI (Polyetherimid), PPA (Polyphthalamid), PPS (Polyphenylensulfid) oder eine Mischung von zwei oder mehr dieser Kunststoffe auf, beispielsweise eine Mischung von PC und ABS. Der Kunststoffkörper kann ferner Vertärkungsfasern aufweisen, beispielsweise Glas-, Basalt-, Bor-, Keramik-, Kieselsäure-, Aramid-, Kohlenstoff-, Polyester-, Nylon-, Polyethylen- oder Plexiglas-Fasern.

Die Metallschicht kann aufgrund ihres Materials, ihrer Oberflächenstruktur oder mittels einer weiteren Antireflexionsschicht so ausgebildet sein, dass störende helle Reflexe des Beleuchtungslichts an dem medizinischen Greifwerkzeug vermindert oder verhindert werden.

### Kurzbeschreibung der Figuren

Nachfolgend werden Ausführungsformen anhand der beigefügten Figuren näher erläutert. Es zeigen:
- Figur 1: eine schematische Darstellung eines medizinischen Greifwerkzeugs;
- Figur 2: eine schematische Darstellung eines weiteren medizinischen Greifwerkzeugs;
- Figur 3: eine schematische Darstellung eines weiteren medizinischen Greifwerkzeugs;
- Figur 4: eine schematische Darstellung eines weiteren medizinischen Greifwerkzeugs;
- Figur 5: eine schematische Darstellung eines weiteren medizinischen Greifwerkzeugs;
- Figur 6: eine schematische Darstellung eines weiteren medizinischen Greifwerkzeugs;
- Figur 7: eine schematische Darstellung eines weiteren medizinischen Greifwerkzeugs;
- Figur 8: ein schematisches Flussdiagramm eines Verfahrens zum Handhaben eines medizinischen Objekts.

### Beschreibung der Ausführungsformen

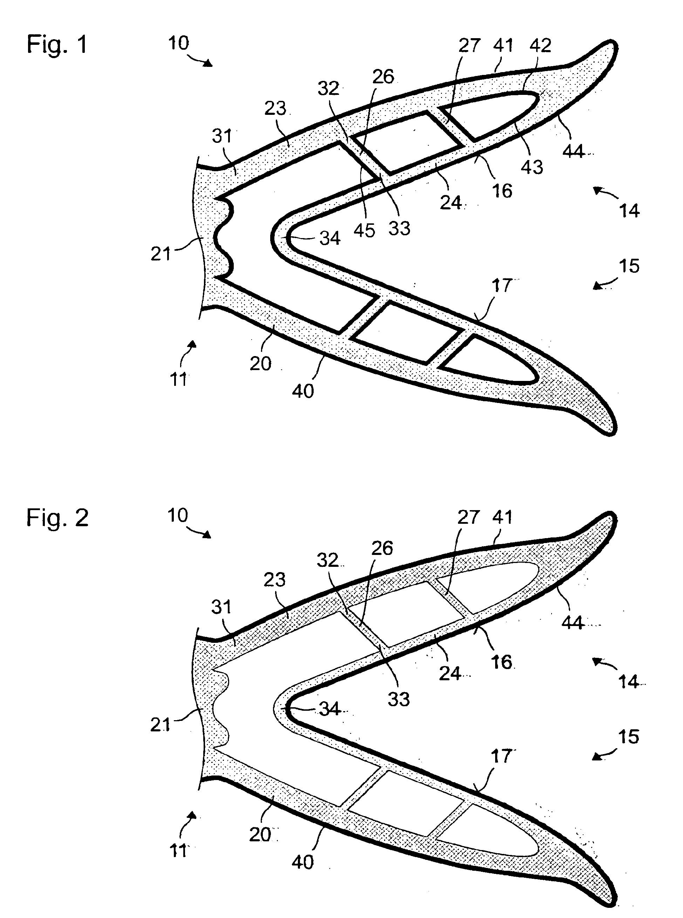

Figur 1 zeigt eine schematische Darstellung eines Schnitt durch ein medizinisches Greifwerkzeug 10. Ein proximales Ende 11 des medizinischen Greifwerkzeugs 10 ist beispielsweise (zerstörungsfrei) lösbar oder dauerhaft bzw. nicht zerstörungsfrei lösbar mit einem distalen Ende eines in Figur 1 nicht dargestellten Schafts mechanisch verbunden. Das medizinische Greifwerkzeug 10 umfasst eine erste Backe 14 und eine zweite Backe 15, die am proximalen Ende 11 des medizinischen Greifwerkzeugs miteinander verbunden sind.

Jede der Backen 14, 15 weist eine Greiffläche 16, 17 auf. Die Greiffläche 16 an der ersten Backe 14 ist der zweiten Backe 15 zugewandt, die Greiffläche 17 an der zweiten Backe 15 ist der ersten Backe 14 zugewandt. Wie nachfolgend beschrieben wird, können die Backen 14, 15 ausgehend von der in Figur 1 dargestellten Position aufeinander zu bewegt werden, bis die Greifflächen 16, 17 einander berühren.

Das medizinische Greifwerkzeug umfasst einen Kunststoffkörper 20, wobei die erste Backe 14 und die zweite Backe 15 jeweils einen Teil des Kunststoffkörpers 20 umfassen. Die erste Backe 14 umfasst einen äußeren Balken 23 und einen inneren Balken 24 des Kunststoffkörpers 20, die distal ineinander übergehen. Nachfolgend wird mit den Attributen "äußerer" und "innerer" auf von den Greifflächen 16, 17 abgewandte bzw. den Greifflächen 16, 17 zugewandte Merkmale oder Seiten Bezug genommen.

Zwischen dem äußeren Balken 23 und dem inneren Balken 24 der ersten Backe 14 sind ein erster Steg 26 und ein zweiter Steg 27 angeordnet. Der erste Steg 26 und der zweite Steg 27 sind zueinander parallel oder im Wesentlichen parallel. Der erste Steg 26 und der zweite Steg 27 können jeweils zum äußeren Balken 23 und zum inneren Balken 24 der ersten Backen 14 senkrecht oder, wie in Figur 1 gezeigt, in einem anderen Winkel angeordnet sein.

Die zweite Backe 15 kann spiegelbildlich oder im Wesentlichen spiegelbildlich zur ersten Backe 14 ausgebildet sein und wird deshalb nachfolgend nicht näher beschrieben. Der äußere Balken 23 der ersten Backe 14 und der entsprechende äußere Balken der zweiten Backe 15 gehen am proximalen Ende 11 des Greifwerkzeugs 10 in ein proximales Ende 21 des Kunststoffkörpers 20 über, über das sie miteinander mechanisch verbunden sind. Die proximalen Enden des inneren Balkens 24 der ersten Backe 14 und des entsprechenden inneren Balkens der zweiten Backe 15 sind nicht unmittelbar mit dem proximalen Ende 21 des Kunststoffkörpers 20 verbunden.

Ein Bereich am proximalen Ende des äußeren Balkens 23 der ersten Backe 14 bzw. ein Übergangsbereich zwischen dem proximalen Ende 21 des Kunststoffkörpers 20 einerseits und dem äußeren Balken 23 der ersten Backe 14 bildet ein erstes Festkörpergelenk 31. Ein an den äußeren Balken 23 der ersten Backe 14 angrenzender Bereich des ersten Stegs 26 bzw. ein Übergangsbereich zwischen dem ersten Steg 26 und dem äußeren Balken 23 bildet ein zweites Festkörpergelenk 32. Ein an den inneren Balken 24 angrenzender Bereich des ersten Stegs 26 bzw. ein Übergangsbereich zwischen dem ersten Steg 26 und dem inneren Balken 24 bildet ein drittes Festkörpergelenk. Am zweiten Steg 27 sind entsprechende Festkörpergelenke vorgesehen, die nicht mit eigenen Bezugszeichen versehen sind. Ein Übergangsbereich zwischen dem inneren Balken 24 der ersten Backe 14 und dem entsprechenden inneren Balken der zweiten Backe 15 bildet ein viertes Festkörpergelenk 34. Abweichend von der Darstellung in Figur 1 können in den Bereichen der Festkörpergelenke 31, 32, 33, 34 verminderte Querschnitte vorliegen, um eine elastische Verformung zu lokalisieren.

An der Oberfläche des Kunststoffkörpers 20 ist eine Metallschicht 40 vorgesehen. Die Metallschicht 40 kann eine einzige Schicht oder ein Mehrschichtsystem aus mehreren Teilschichten umfassen. Beispielsweise umfasst die Metallschicht 40 eine Nickelschicht mit einer mittleren Korngröße von weniger als 100 nm, insbesondere mit einer mittleren Korngröße im Bereich von 10 nm bis 20 nm, und eine Kupferschicht zwischen dem Kunststoffkörper 20 und der Nickelschicht.

Die Metallschicht 40 umfasst mehrere Bereiche, die, wie in Figur 1 dargestellt, ineinander übergehen oder, abweichend von der Darstellung in Figur 1, voneinander durch Lücken getrennt sein können. Ein erster Abschnitt bzw. Bereich 41 der Metallschicht 40 ist an der äußeren Oberfläche des äußeren Balkens 23 vorgesehen. Ein zweiter Abschnitt bzw. Bereich 42 der Metallschicht 40 ist an der inneren Oberfläche des äußeren Balkens 23 angeordnet. Ein dritter Abschnitt bzw. Bereich 43 der Metallschicht 40 ist an der äußeren Oberfläche des inneren Balkens 24 angeordnet. Ein vierter Abschnitt bzw. Bereich 44 der Metallschicht 40 ist an der inneren Oberfläche des inneren Balkens 24 bzw. an der Greiffläche 16 der ersten Backe 14 angeordnet. Ein fünfter Bereich 45 der Metallschicht 40 ist an der Oberfläche des ersten Stegs 24 angeordnet. Entsprechende Bereiche der Metallschicht 40 sind am zweiten Steg 27 und an der zweiten Backe 15 angeordnet.

Der Kunststoffkörper 20 und die Metallschicht 40 sind elastisch verformbar, insbesondere in den Bereichen der Festkörpergelenke 31, 32, 33, 34. Da der Kunststoffkörper 22 eine höhere Elastizität aufweisen kann als die Metallschicht 40, kann das medizinische Greifwerkzeug 10 so ausgebildet sein, dass bei der vorgesehenen Verwendung der Kunststoffkörper 20 elastisch und die Metallschicht 40 teilweise nur elastisch und teilweise auch plastisch verformt werden. Insbesondere kann das medizinische Greifwerkzeug 10 so ausgebildet sein, dass die Metallschicht 40 in den Bereichen der Festkörpergelenke 31, 32, 33, 34 bei der vorgesehenen Verwendung plastisch verformt wird.

Das medizinische Greifwerkzeug 10 ist insbesondere so verformbar, dass die Backen 14, 15 aufeinander zu bewegt werden bis die Greifflächen 16, 17 einander oder einen zwischen ihnen angeordneten medizinischen Gegenstand berühren. Dazu wird insbesondere das vierte Festkörpergelenk 34 zwischen den inneren Balken 24 beider Backen 14, 15 mittels einer in Figur 1 nicht dargestellten Einrichtung nach proximal gezogen. Dabei können sich die Backen 14, 15 entsprechend dem Flossenstrahlen- bzw. Fin-Ray-Effekt verformen. Das medizinische Greifwerkzeug 10 bzw. dessen Backen 14, 15 können insbesondere so ausgebildet sein, dass sie beim Greifen eines konvexen Gegenstands diesen jeweils bogenförmig umschließen.

Wie bereits erwähnt, beeinflusst die Metallschicht 40 die mechanischen Eigenschaften der Backen 14, 15, insbesondere ihre Elastizität und ihre Verformung beim Greifen eines medizinischen Gegenstands. Die Metallschicht 40 beeinflusst dabei die mechanischen Eigenschaften des medizinischen Greifwerkzeugs 10 an unterschiedlichen Stellen in unterschiedlicher Weise. Abweichend von der Darstellung in Figur 1 kann die Metallschicht 40 in verschiedenen Bereichen verschiedene Eigenschaften aufweisen, beispielsweise unterschiedliche Schichtaufbauten, Materialien, Schichtdicken. Ferner kann die Metallschicht 40 elektrische Funktionen erfüllen und dazu in verschiedenen Bereichen des medizinischen Greifwerkzeugs 10 unterschiedlich ausgebildet sein.

Nachfolgend werden anhand der Figuren 2 bis 5 weitere Ausführungsformen des medizinischen Greifwerkzeugs 10 dargestellt. Dabei sind ähnlich wie in Figur 1 jeweils beide Backen 14, 15 symmetrisch dargestellt. Abweichend davon können die beiden Backen 14, 15 jedoch sowohl hinsichtlich des Kunststoffkörpers 20 als auch hinsichtlich der Metallschicht 40 unterschiedlich ausgebildet sein.

Figur 2 zeigt eine schematische Darstellung eines medizinischen Greifwerkzeugs 10, das in einigen Merkmalen dem oben anhand der Figur 1 dargestellten medizinischen Greifwerkzeug ähnelt, insbesondere hinsichtlich des Kunststoffkörpers 20. Im Unterschied zu dem oben anhand der Figur 1 dargestellten medizinischen Greifwerkzeug sind jedoch bei dem in Figur 2 dargestellten medizinischen Greifwerkzeug 10 lediglich der erste Bereich 41 der Metallschicht 40 an der äußeren Oberfläche des äußeren Balkens 23 und der vierte Bereich 44 der Metallschicht 40 an den Greifflächen 16, 17 vorgesehen. An der inneren Oberfläche des äußeren Balkens 23, an der äußeren Oberfläche des inneren Balkens 24 und an den Stegen 26, 27 ist keine Metallschicht vorgesehen. Durch diese Ausbildung des medizinischen Greifwerkzeugs 10 kann beispielsweise eine besonders hohe Elastizität der Stege 26, 27 und gleichzeitig eine besonders hohe Zug-Steifheit der Balken 23, 24 erzeugt werden.

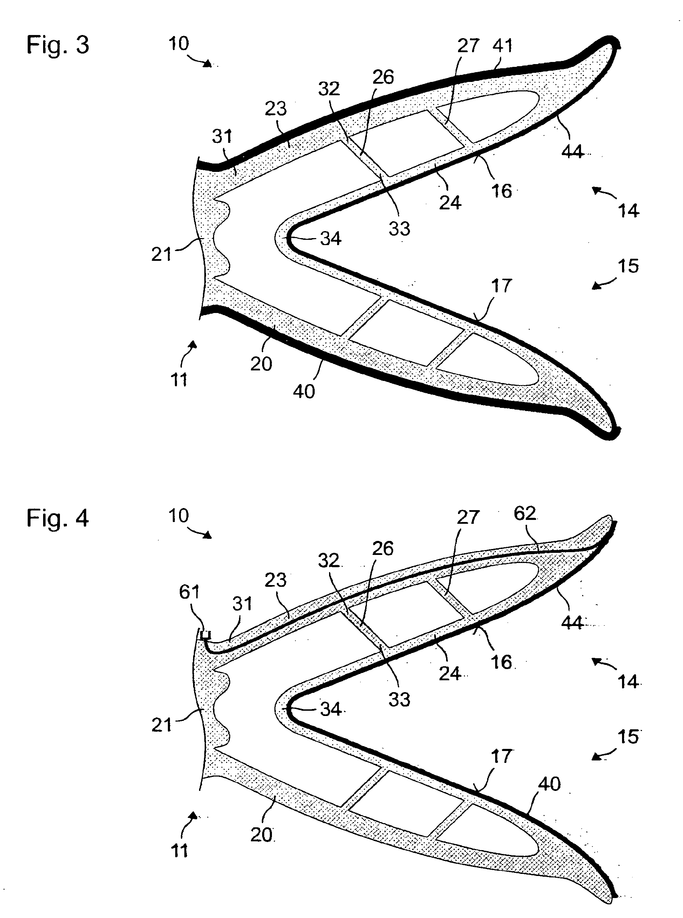

Figur 3 zeigt eine schematische Darstellung eines weiteren medizinischen Greifwerkzeugs 10, das in einigen Merkmalen dem oben anhand der Figur 2 dargestellten medizinischen Greifwerkzeug ähnelt. Im Unterschied zu diesem ist bei dem in Figur 3 dargestellten medizinischen Greifwerkzeug 10 die Metallschicht 40 im ersten Bereich 41 an der äußeren Oberfläche des äußeren Balkens 23 dicker ausgeführt als im vierten Bereich 44 an der inneren Oberfläche des inneren Balkens 24. Durch diese Ausgestaltung kann beispielsweise die Biegeelastizität des inneren Balkens 24 höher oder wesentlich höher sein als die des äußeren Balkens 23. Zu diesem Zweck kann abweichend von der Darstellung in Figur 3 die Metallschicht 40 im vierten Bereich 44 an der Greiffläche 16 ganz entfallen. Mit der in Figur 3 dargestellten dünnen Metallschicht im vierten Bereich 44 an der inneren Oberfläche des inneren Balkens 24 kann gleichzeitig eine ausreichende Elastizität des inneren Balkens 24 gewährleistet und eine elektrische Funktionalität ermöglicht werden.

Figur 4 zeigt eine schematische Darstellung eines weiteren medizinischen Greifwerkzeugs 10, das in einigen Merkmalen dem oben anhand der Figur 1 dargestellten medizinischen Greifwerkzeug und insbesondere den oben anhand der Figuren 2 und 3 dargestellten medizinischen Greifwerkzeugen ähnelt. Im Unterschied zu den oben anhand der Figuren 1 bis 3 dargestellten medizinischen Greifwerkzeugen weist das in Figur 4 gezeigte medizinische Greifwerkzeug 10 lediglich an den Greifflächen 16, 17 eine Metallschicht 40 auf. Diese Metallschicht 40 im vierten Bereich 44 an den Greifflächen 16,17 ermöglicht insbesondere eine elektrische Funktionalität und/oder eine verringerte Elastizität, insbesondere eine verringerte Dehn- bzw. Stauchbarkeit- des inneren Balkens 24.

Im Unterschied zu den oben anhand der Figuren 1 bis 3 dargestellten medizinischen Greifwerkzeugen weist das in Figur 4 dargestellte Greifwerkzeug 10 ferner einen elektrischen Steckkontakt 61 am distalen Ende 11 und einen Draht oder eine Litze 62 auf. Der Draht oder die Litze 62 ist in den Kunststoffkörper 20 eingebettet, insbesondere eingegossen, und verbindet den elektrischen Steckkontakt 61 elektrisch leitfähig mit der Metallschicht 40. Dazu liegt der Draht bzw. die Litze 62 am distalen Ende der Backe 14 an der Greiffläche 16 und berührt dort die Metallschicht 40. Ferner kann der Draht bzw. die Litze 62 ausgebildet sein, um die mechanischen Eigenschaften des Kunststoffkörpers, insbesondere des äußeren Balkens 23, zu beeinflussen.

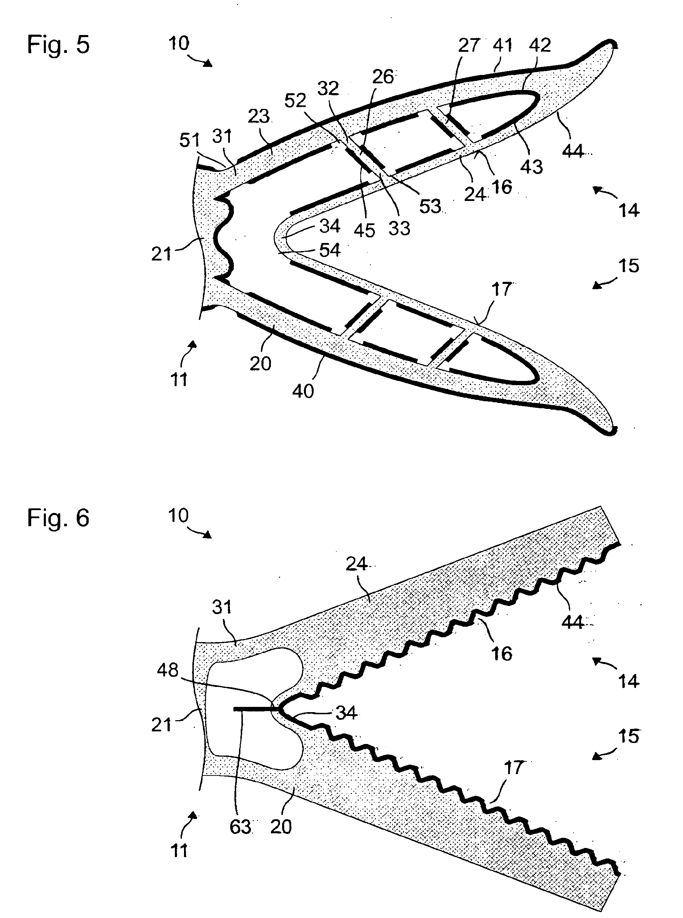

Figur 5 zeigt eine schematische Darstellung eines medizinischen Greifwerkzeugs 10, das insbesondere dem oben anhand der Figur 1 dargestellten medizinischen Greifwerkzeug in einigen Merkmalen ähnelt. Im Unterschied zu dem oben anhand der Figur 1 dargestellten medizinischen Greifwerkzeug weist das in Figur 5 dargestellte medizinische Greifwerkzeug 10 an den Greifflächen 16, 17 keine Metallschicht auf. Abweichend von der Darstellung in Figur 5 kann jedoch alternativ an den Greifflächen 16, 17 eine Metallschicht vorgesehen sein, die dünner sein kann als in den anderen Bereichen 41, 42, 43, 45.

Ferner unterscheidet sich das in Figur 5 dargestellte medizinische Greifwerkzeug 10 von dem oben anhand der Figur 1 dargestellten medizinischen Greifwerkzeug dadurch, dass die Metallschicht 40 in den Bereichen der Festkörpergelenke 31, 32, 33, 34 Aussparungen bzw. Lücken 51, 52, 53, 54 aufweist. Dadurch kann die Elastizität des Kunststoffkörpers im Bereich der Festkörpergelenke 31, 32, 33, 34 ungeschmälert erhalten bleiben. In anderen Bereichen reduziert die Metallschicht 40 die Elastizität des medizinischen Greifwerkzeugs 10. Dadurch ist die Verformung des medizinischen Greifwerkzeugs 10 ausgeprägter als bei dem oben anhand der Figur 1 dargestellten medizinischen Greifwerkzeug und ausgeprägter als bei einem medizinischen Greifwerkzeug ganz ohne eine Metallschicht auf die Bereiche der Festkörpergelenke 31, 32, 33, 34 lokalisiert. Die kinematischen Eigenschaften des medizinischen Greifwerkzeugs 10 sind deshalb besser definiert bzw. unabhängiger von den Kräften und Momenten, die beim Greifen eines realen Gegenstands auftreten.

Figur 6 zeigt eine schematische Darstellung eines weiteren medizinischen Greifwerkzeugs 10 mit einer ersten Backe 14 und einer zweiten Backe 15. Im Unterschied zu den oben anhand der Figuren 1 bis 5 dargestellten medizinischen Greifwerkzeugen weist das in Figur 6 dargestellte medizinische Greifwerkzeug 10 an den Greifflächen 16, 17 jeweils Zähne oder eine Riffelung 18 auf. Im weiteren Unterschied zu den oben anhand der Figuren 1 bis 5 dargestellten medizinischen Greifwerkzeugen weisen die Backen 14, 15 des in Figur 6 dargestellten medizinischen Greifwerkzeugs 10 keine Fachwerk-Struktur auf. Am proximalen Ende sind die Backen 14, 15 über Festkörpergelenke 36, 37, 38 miteinander verbunden. Indem das mittlere Festkörpergelenk 37, das die Greifflächen 16, 17 der Backen 14, 15 unmittelbar miteinander verbindet, nach proximal gezogen wird, können die Backen 14, 15 auf einander zu bewegt werden.

Ähnlich wie bei dem oben anhand der Figur 4 dargestellten medizinischen Greifwerkzeug ist nur eine Metallschicht 44 an den Greifflächen 16, 17 des medizinischen Greifwerkzeugs 10 vorgesehen. Diese Metallschicht 44 kann die mechanische Steifheit der Backen 14, 15 erhöhen und/oder eine elektrische Funktionalität ermöglichen. Abweichend von der Darstellung in Figur 6 kann die Metallschicht beispielsweise an der gesamten Oberfläche der Backen 14, 15 oder nur an den Außenseiten der Backen 14, 15 vorgesehen sein.

Die Metallschicht 44 ist mittels einer Leiterbahn 48 elektrisch leitfähig mit einer Lötfahne 63 verbunden. Die Leiterbahn 48 kann Bestandteil der Metallschicht 40 und insbesondere gleichzeitig mit dieser hergestellt sein. Über die Lötfahne 63 kann die Metallschicht kontaktiert werden, beispielsweise um eine Wechselspannung geeigneter Frequenz und Amplitude zur Koagulation von Gewebe anzulegen.

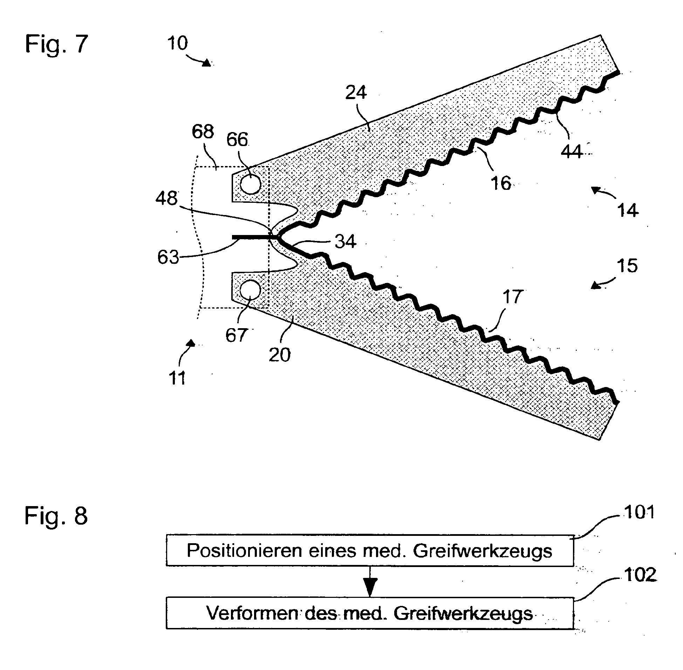

Figur 7 zeigt eine schematische Darstellung eines medizinischen Greifwerkzeugs 10, das in einigen Merkmalen dem oben anhand der Figur 6 dargestellten medizinischen Greifwerkzeug ähnelt. Im Unterschied zu dem oben anhand der Figur 6 dargestellten medizinischen Greifwerkzeug weist das in Figur 7 dargestellte medizinische Greifwerkzeug 10 anstelle zweier Festkörpergelenke herkömmliche Gelenke mit Wellen 66, 68 auf. Über die Wellen 66,67 sind die Backen 14,15 schwenkbar bzw. drehbar mit einem distalen Ende 68 eines in Figur 7 nur angedeuteten Schafts eines medizinischen Instruments verbunden. Das medizinische Greifwerkzeug 10 kann das medizinische Instrument sein oder Teil des medizinischen Instruments sein.

Wie bei dem oben anhand der Figur 6 dargestellten Greifwerkzeug ist auch bei dem in Figur 7 dargestellten Greifwerkzeug 10 die Metallschicht 40 mittels einer Leiterbahn 48 elektrisch leitfähig mit einer Lötfahne 63 verbunden. Jedes der beiden anhand der Figuren 6 und 7 dargestellten medizinischen Greifwerkzeuge kann anstelle einer Lötfahne einen elektrischen Steckkontakt oder eine beliebige andere Kontaktierungseinrichtung aufweisen.

Jedes der oben anhand der Figuren 1 bis 3 und 5 bis 7 dargestellten medizinischen Greifwerkzeuge kann einen Steckkontakt, wie er anhand der Figur 4 dargestellt wurde, eine Lötfahne, wie sie anhand der Figuren 6 und 7 dargestellt wurden, oder andere Kontaktierungseinrichtungen aufweisen. Bei einem medizinischen Greifwerkzeug, das, wie oben anhand der Figuren 1 bis 3 dargestellt, eine von den Greifflächen 16, 17 bis zum proximalen Ende 11 durchgehende Metallschicht aufweist, kann diese am proximalen Ende 11 beispielsweise mittels einer Klemmung kontaktiert werden.

Die oben anhand der Figuren 1 bis 5 dargestellten medizinischen Greifwerkzeuge können ähnlich wie die oben anhand der Figuren 6 und 7 dargestellten Greifwerkzeuge Riffelungen an den Greifflächen 16, 17 aufweisen. Die oben anhand der Figuren 6 und 7 dargestellten medizinischen Greifwerkzeuge können ähnlich wie die oben anhand der Figuren 1 bis 5 dargestellten Greifwerkzeuge glatte oder im Wesentlichen glatte Greifflächen 16, 17 aufweisen. Bei den oben anhand der Figuren 1 bis 5 dargestellten medizinischen Greifwerkzeugen können ähnlich wie bei dem anhand der Figur 7 dargestellten medizinischen Greifwerkzeug die Festkörpergelenke teilweise durch herkömmliche Gelenke mit Wellen ersetzt werden.

Figur 8 zeigt ein schematisches Flussdiagramm eines Verfahrens zum Handhaben eines medizinischen Objekts. Das Verfahren ist insbesondere mit medizinischen Greifwerkzeugen ausführbar, wie sie oben anhand der Figuren 1 bis 7 dargestellt wurden. Das Verfahren ist jedoch auch mit medizinischen Greifwerkzeugen ausführbar, die sich von den oben anhand der Figuren 1 bis 7 dargestellten unterscheiden. Die nachfolgende Verwendung von Bezugszeichen aus den Figuren 1 bis 7 dient deshalb lediglich exemplarisch der Verbesserung des Verständnisses.

Bei einem ersten Schritt 101 wird ein medizinisches Greifwerkzeug 10 mit einem Kunststoffkörper 20 und einer Metallschicht 40 an einer Oberfläche des Kunststoffkörpers 20 an dem medizinischen Objekt positioniert. Bei einem zweiten Schritt 102 wird das medizinische Greifwerkzeug verformt, um das medizinische Objekt zu greifen, wobei der Kunststoffkörper zumindest elastisch verformt wird. Dabei kann die Metallschicht 40 teilweise elastisch, teilweise plastisch verformt werden. Die Verformung beim zweiten Schritt 102 ist insbesondere eine nicht nur unwesentliche, wie sie insbesondere an Festkörpergelenken auftritt. Beim zweiten Schritt 102 wird das Greifwerkzeug insbesondere entsprechend dem Flossenstrahlen-Effekt verformt.

### Bezugszeichen

- 10: medizinisches Greifwerkzeug
- 11: proximales Ende des medizinischen Greifwerkzeugs 10
- 14: erste Backe des medizinischen Greifwerkzeugs 10
- 15: zweite Backe des medizinischen Greifwerkzeugs 10
- 16: Greiffläche an der ersten Backe 14
- 17: Greiffläche an der zweiten Backe 15
- 20: Kunststoffkörper
- 21: proximales Ende des Kunststoffkörpers 20
- 23: äußerer Balken der ersten Backe 14
- 24: innerer Balken der ersten Backe 14
- 26: erster Steg zwischen dem äußeren Balken 23 und dem inneren Balken 24
- 27: zweiter Steg zwischen dem äußeren Balken 23 und dem inneren Balken 24
- 31: erstes Festkörpergelenk zwischen dem proximalem Ende 21 und dem äußeren Balken 23
- 32: zweites Festkörpergelenk zwischen dem äußeren Balken 23 und dem ersten Steg 26
- 33: drittes Festkörpergelenk zwischen dem inneren Balken 24 und dem ersten Steg 26
- 34: viertes Festkörpergelenk zwischen den inneren Balken 24 beider Backen 14, 15
- 36: erstes Festkörpergelenk
- 37: zweites Festkörpergelenk
- 38: drittes Festkörpergelenk
- 40: Metallschicht auf Oberfläche des Kunststoffkörpers 20
- 41: Metallschicht bzw. erster Bereich der Metallschicht 40 an äußerer Oberfläche des äußeren Balkens 23
- 42: Metallschicht bzw. zweiter Bereich der Metallschicht 40 an innerer Oberfläche des äußeren Balkens 23
- 43: Metallschicht bzw. dritter Bereich der Metallschicht 40 an äußerer Oberfläche des inneren Balkens 24
- 44: Metallschicht bzw. vierter Bereich der Metallschicht 40 an Greiffläche 16
- 45: fünfter Bereich Metallschicht 40 an Oberfläche des ersten Stegs 26
- 48: Leiterbahn
- 51: Lücke in der Metallschicht 30 im Bereich des ersten Festkörpergelenks 31
- 52: Lücke in der Metallschicht 30 im Bereich des zweiten Festkörpergelenks 32
- 53: Lücke in der Metallschicht 30 im Bereich des dritten Festkörpergelenks 33
- 54: Lücke in der Metallschicht 30 im Bereich des vierten Festkörpergelenks 34
- 61: elektrischer Steckkontakt
- 62: Draht im Kunststoffkörper 20
- 63: Lötfahne
- 66: erste Welle
- 67: zweite Welle
- 68: distales Ende eines Schafts eines medizinischen Instruments
- 101: erster Schritt
- 102: zweiter Schritt

## Patentansprüche

1. **Medizinisches Greifwerkzeug** (10), mit:
einem **Kunststoffkörper** (20) mit einer ersten Backe (14) und einer zweiten Backe (15), der dazu ausgebildet ist, bei seiner vorgesehenen Verwendung verformt zu werden; **dadurch gekennzeichnet dass**,
eine **Metallschicht** (40) an einer Oberfläche des Kunststoffkörpers (20) im Bereich zumindest entweder der ersten Backe (14) oder der zweiten Backe (15) vorgesehen ist.

2. Medizinisches Greifwerkzeug (10) nach dem vorangehenden Anspruch, wobei das Greifwerkzeug (10) dazu ausgebildet ist, dass die Metallschicht (40) bei der vorgesehenen Verwendung plastisch verformt wird.

3. Medizinisches Greifwerkzeug (10) nach einem der vorangehenden Ansprüche, bei dem der Kunststoffkörper (20) ein **Festkörpergelenk** (34; 37) zwischen der ersten Backe (14) und der zweiten Backe (15) umfasst.

4. Medizinisches Greifwerkzeug (10) nach einem der vorangehenden. Ansprüche, bei dem zumindest entweder die erste Backe (14) oder die zweite Backe (15) eine **Fachwerk-**Struktur aufweisen.

5. Medizinisches Greifwerkzeug (10) nach dem vorangehenden Anspruch, bei dem zumindest entweder die erste Backe (14) oder die zweite Backe (15) ausgebildet ist, um sich beim Greifen eines Gegenstands entsprechend dem **Flossenstrahlen-Effekt** zu verformen.

6. Medizinisches Greifwerkzeug (10) nach einem der vorangehenden Ansprüche, bei dem die Metallschicht (40) eine mittlere **Korngröße** von höchstens 100 nm aufweist

7. Medizinisches Greifwerkzeug (10) nach einem der vorangehenden Ansprüche, bei dem die Metallschicht (40) an einer **Greifflächen** (16, 17) des Greifwerkzeugs (10) vorgesehen ist.

8. Medizinisches Greifwerkzeug (10) nach einem der vorangehenden Ansprüche, bei dem an einer **Greiffläche** (16, 17) des Greifwerkzeugs (10) **keine** Metallschicht (40) oder eine **dünnere** Metallschicht (40) als an anderen Bereichen der Oberfläche des Kunststoffkörpers (20) vorgesehen ist.

9. Medizinisches Greifwerkzeug (10) nach einem der vorangehenden Ansprüche, bei dem an einem **Festkörpergelenk** (31, 32, 33, 34) des Greifwerkzeugs **keine** Metallschicht (40) oder eine **dünnere** Metallschicht (40) als an anderen Bereichen der Oberfläche des Kunststoffkörpers (20) vorgesehen ist.

10. Medizinisches Greifwerkzeug (10) nach einem der vorangehenden Ansprüche, ferner mit:
einer **Kontaktierungseinrichtung** (61; 63) zum elektrischen Kontaktieren der Metallschicht (40),
wobei die Metallschicht (40) einen Abschnitt an einer Greiffläche (16, 17) umfasst, wobei der Abschnitt an der Greiffläche (16, 17) elektrisch leitfähig mit der Kontaktierungseinrichtung (61; 63) verbunden ist.

11. Medizinisches Greifwerkzeug (10) nach einem der vorangehenden Ansprüche, bei der die Metallschicht (40) eine Nickelschicht mit einer mittleren Korngröße von höchstens 100 nm und eine Kupferschicht zwischen dem Kunststoffkörper (20) und der Nickelschicht umfasst.

## Claims

1. Medical gripping tool (10), comprising:
a plastic body (20) with a first jaw (14) and a second jaw (15), which plastic body (20) is designed to be deformed in its intended use; **characterized in that**
a metal layer (40) is provided on a surface of the plastic body (20) in the area of at least either the first jaw (14) or the second jaw (15).

2. Medical gripping tool (10) according to the preceding claim, wherein the gripping tool (10) is designed such that the metal layer (40) is plastically deformed in the intended use.

3. Medical gripping tool (10) according to one of the preceding claims, in which the plastic body (20) comprises a solid joint (34; 37) between the first jaw (14) and the second jaw (15).

4. Medical gripping tool (10) according to one of the preceding claims, in which at least either the first jaw (14) or the second jaw (15) has a truss structure.

5. Medical gripping tool (10) according to the preceding claim, in which at least either the first jaw (14) or the second jaw (15) is designed to deform according to the fin ray effect when gripping an object.

6. Medical gripping tool (10) according to one of the preceding claims, in which the metal layer (40) has an average grain size of at most 100 nm.

7. Medical gripping tool (10) according to one of the preceding claims, in which the metal layer (40) is provided on a grip surface (16, 17) of the gripping tool (10).

8. Medical gripping tool (10) according to one of the preceding claims, in which no metal layer (40) is provided on a grip surface (16, 17) of the gripping tool (10), or a metal layer (40) is provided thereon that is thinner than on other areas of the surface of the plastic body (20).

9. Medical gripping tool (10) according to one of the preceding claims, in which no metal layer (40) is provided on a solid joint (31, 32, 33, 34) of the gripping tool, or a metal layer (40) is provided thereon that is thinner than on other areas of the surface of the plastic body (20).

10. Medical gripping tool (10) according to one of the preceding claims, moreover comprising:
a contacting device (61; 63) for electrically contacting the metal layer (40);
wherein the metal layer (40) comprises a portion on a grip surface (16, 17), wherein the portion on the grip surface (16, 17) is connected to the contacting device (61; 63) in an electrically conductive manner.

11. Medical gripping tool (10) according to one of the preceding claims, in which the metal layer (40) comprises a nickel layer with an average grain size of at most 100 nm, and a copper layer between the plastic body (20) and the nickel layer.

## Revendications

1. Outil de préhension médical (10), avec un corps en matière plastique (20) avec une première mâchoire (14) et une deuxième mâchoire (15), qui est conçu pour être déformé lors de son utilisation prévue, **caractérisé en ce qu'**il est prévu une couche métallique (40) sur une surface du corps en matière plastique (20) dans la région d'au moins soit la première mâchoire (14) soit la deuxième mâchoire (15).

2. Outil de préhension médical (10) selon la revendication précédente, dans lequel l'outil de préhension (10) est conçu de telle manière que la couche métallique (40) soit déformée plastiquement lors de l'utilisation prévue.

3. Outil de préhension médical (10) selon l'une quelconque des revendications précédentes, dans lequel le corps en matière plastique (20) comprend une articulation à corps solide (34; 37) entre la première mâchoire (14) et la deuxième mâchoire (15).

4. Outil de préhension médical (10) selon l'une quelconque des revendications précédentes, dans lequel au moins soit la première mâchoire (14) soit la deuxième mâchoire (15) présente une structure en treillis.

5. Outil de préhension médical (10) selon la revendication précédente, dans lequel au moins soit la première mâchoire (14) soit la deuxième mâchoire (15) est conçue pour se déformer lors de la saisie d'un objet selon l'effet de queue de poisson.

6. Outil de préhension médical (10) selon l'une quelconque des revendications précédentes, dans lequel la couche métallique (40) présente une taille de grain moyenne de 100 nm au maximum.

7. Outil de préhension médical (10) selon l'une quelconque des revendications précédentes, dans lequel la couche métallique (40) est prévue sur une face de saisie (16, 17) de l'outil de préhension (10).

8. Outil de préhension médical (10) selon l'une quelconque des revendications précédentes, dans lequel il est prévu sur une face de saisie (16, 17) de l'outil de préhension (10) soit aucune couche métallique (40) soit une couche métallique (40) plus mince que sur d'autres régions de la surface du corps en matière plastique (20).

9. Outil de préhension médical (10) selon l'une quelconque des revendications précédentes, dans lequel il est prévu sur une articulation à corps solide (31, 32, 33, 34) de l'outil de préhension soit aucune couche métallique (40) soit une couche métallique (40) plus mince que sur d'autres parties de la surface du corps en matière plastique (20).

10. Outil de préhension médical (10) selon l'une quelconque des revendications précédentes, avec en outre un dispositif de mise en contact (61; 63) pour la mise en contact électrique de la couche métallique (40), dans lequel la couche métallique (40) comprend une partie sur une face de saisie (16, 17), dans lequel la partie sur la face de saisie (16, 17) est reliée de façon électriquement conductrice au dispositif de mise en contact (61; 63).

11. Outil de préhension médical (10) selon l'une quelconque des revendications précédentes, dans lequel la couche métallique (40) comprend une couche de nickel avec une taille de grain moyenne de 100 nm au maximum et une couche de cuivre entre le corps en matière plastique (20) et la couche de nickel.
